# EUROPEAN PATENT APPLICATION

(11) **EP 0 911 023 A1**
(43) Date of publication of application: **28.04.1999**
(21) Application number: 98830158.6
(22) Date of filing: 20.03.1998
(51) Int. Cl.: A61K 9/00, A61K 9/127, A61K 9/48, A61K 7/00

(54) **Pharmaceutical or cosmetic compositions containing photosensitizing substances**

(71) Applicant: Russo, Mario Luca, 31021 Mogliano Veneto (Treviso) (IT); Casale, Antonio, 35020 Casalserugo (Padova) (IT)
(72) Inventor: Russo, Mario Luca, 31021 Mogliano Veneto (Treviso) (IT); Casale, Antonio, 35020 Casalserugo (Padova) (IT)
(74) Representative: Ferreccio, Rinaldo

(57) **Abstract**

Liposomes containing at least one pharmaceutically or cosmetically active ingredient and a photosensitizing substance showing a high degree of absorption at at least one wavelength of a light source, cosmetic or pharmaceutical compositions which contain the liposomes, and a treatment method which uses these compositions; preferably, the photosensitizing substance is indocyanine and the light source is a laser beam emitted by a power diode laser with a wavelength of between 700 and 900 nm.

## Description

The present invention relates in general to pharmaceutical or cosmetic compositions containing photosensitising substances.

In particular, the invention relates to pharmaceutical or cosmetic compositions comprising photosensitizing substances, possibly enclosed in heat-sensitive liposomes, and to cosmetic or therapeutic methods of treatment based on these compositions.

It is known that liposomes are used to an ever greater extent in cosmetic and pharmaceutical formulations for topical use owing to their ability to promote and increase the absorption of active ingredients through the skin by virtue of their simultaneously hydrophilic and lipophilic characteristics, which enable them to pass through the horny barrier and diffuse through the skin layers, reaching the papillary dermis and possibly the microcirculation.

In the prior art, therapeutic formulations for systemic use containing heat-sensitive liposomes (DSPCs) have also been prepared with the intention of subsequently releasing the active ingredient (for example, an anti-tumour agent) only in a certain anatomical region in which the temperature is raised by heating so as to melt the liposomes and release the active ingredient.

The problem upon which the present invention is based is that of providing cosmetic or pharmaceutical compositions comprising liposomes which can selectively release the active ingredients contained therein only in those anatomical regions in which a cosmetic or therapeutic treatment is to be brought about, as well as increasing the effects of the active ingredients released.

This problem is solved, according to the invention, by the provision of liposomes containing at least one pharmacologically or cosmetically active ingredient, characterized in that they further contain at least one photosensitizing substance showing a high degree of absorption at at least one wavelength of a light source.

The photosensitizing substance is preferably selected from the group comprising haematoporphyrin derivatives, chlorophyll derivatives, indocyanine green, photophrin II, micro-cyanine, and Chlorin-E6 and the light source used is preferably a laser beam.

The following light sources may be used for these photosensitizing substances:
- haematoporphyrin derivatives: argon dye laser (630 nm)
- chlorophyll derivatives: argon dye laser (630 nm)
- photophrin II: argon dye laser (630 nm)
- micro-cyanine: copper vapour (540 nm)
- Chlorin-E6: dye laser (660 nm)
- indocyanine green: diode laser (700-900 nm)

Indocyanine green, which has low toxicity and the pharmacokinetics of which are widely known, is particularly preferred as the photosensitizing substance and a continuous, pulsed, or micro-pulsed power diode laser with a wavelength of between 700 and 900nm is particularly preferred as the light source since most biological structures are transparent at this wavelength. Moreover, the use of a diode laser is particularly advantageous because of its ease of handling and its low production and maintenance costs.

A further aspect of the present invention relates to a cosmetic composition for topical application comprising a cosmetically acceptable vehicle and the liposomes described above, particularly those containing indocyanine green.

Alternatively, the present invention relates to a cosmetic composition for topical application comprising a cosmetically acceptable vehicle and a photosensitizing substance showing a high degree of absorption at at least one wavelength of a light source, selected from the group comprising haematoporphyrin derivatives, chlorophyll derivatives, indocyanine green, photophrin II, micro-cyanine, and Chlorin-E6.

The cosmetic composition according to the invention may be used in a cosmetic treatment method comprising the topical application of the composition and the subsequent irradiation of the application site with a source of light having a wavelength at which the photosensitizing substance shows absorption.

The present invention also relates to a pharmaceutical composition comprising a pharmaceutically acceptable excipient and liposomes such as those described above, particularly those containing indocyanine green.

This pharmaceutical composition may be used in a therapeutic treatment method comprising the topical application of the composition and the subsequent irradiation of the application site with a source of light having a wavelength at which the photosensitizing substance shows absorption.

A further aspect of the invention relates to a pharmaceutical composition comprising a pharmaceutically acceptable excipient and liposomes as described above for use in a therapeutic treatment method comprising the administration of the composition by a systemic route and the subsequent irradiation of one or more anatomical regions which require treatment with a light source having a wavelength at which the photosensitizing substance shows absorption.

The liposomes used in the cosmetic and pharmaceutical compositions for topical use according to the invention are preferably multilamellar and based on phospholipids derived from soya lecithin, whereas those used in pharmaceutical formulations for systemic administration are preferably unilamellar.

The basic principle on which the present invention is based is that of the utilization of the recognized "carrier" properties of liposomes to carry one or more active ingredients and the subsequent achievement of selective release in the regions of the body to be acted on by the application of a source of light of suitable wavelength which is absorbed by the photosensitizing substance contained in the liposomes which in turn is excited and brings about a local rise in temperature causing the liposome to melt and release the active ingredient.

The activity of the active ingredient is also amplified by the local rise in temperature which is itself a catalysing factor.

Particularly significant results can be achieved in the cosmetic field in cases in which it is necessary to bring about absorption of the active ingredient down to the deepest layers of the skin and beyond, as is the case in the treatment of cellulite. In these cases, deep absorption is in fact achieved by virtue of the ability of liposomes to act as vehicles and the effect of the active ingredients is increased by the massive release thereof which is achieved when the region of the skin to be treated is irradiated with a source of light of a wavelength such as to interact with the photosensitizing substance, and by the local rise in temperature in the region treated.

In the therapeutic field, a targeted release of active ingredients exclusively in well-defined areas can be achieved. For example, if a formulation according to the invention comprising liposomes which are stable at normal body temperature and which contain an anti-tumour agent and a photosensitizing substance is administered intravenously, the active ingredient can then be released exclusively in the anatomical region affected by the tumour by the irradiation solely of this region with the wavelength which can interact with the photosensitizing substance.

Further advantages of the liposomes and of the compositions according to the invention will become clearer from the examples given by way of non-limiting example below.

### EXAMPLE 1

Unilamellar liposomes containing indocyanine green as a photosensitizing substance and cytosine arabinoside as an active ingredient were prepared as follows.

The liposomes were produced from 12.5g of a 4:1 mixture by weight of dipalmitoyl lecithin and dipalmitoyl phosphatidyl glycerol. This mixture was dissolved in 400ml of chloroform and 800ml of isopropyl ether.

A HEPES (N-2-hydroxyethyl-piperazine-N'-2-ethane-sulphonic acid) buffer solution (145mM NaCl, 6mM KCl, 10mM HEPES pH 7.4) was prepared separately and diluted 1:10 with distilled water. The cytosine arabinoside was added to the diluted HEPES buffer solution to give a final concentration of 300mM; a quantity of indocyanine green such as to give a final concentration of 300mM was also added and, finally, the pH was adjusted to 7.4 with 0.1 N HCl.

The aqueous solution thus obtained (400 ml) and the organic phospholipid solution (1200ml) were heated to 50°C and combined in an Erlenmeyer flask which was then filled with nitrogen and sealed.

The mixture of the two phases was introduced into an ultrasound bath and sonicated at 45-50°C for 5 minutes to give a homogeneous emulsion. The emulsion was transferred to a rotary-evaporator flask and evaporated with care under a slight vacuum with a bath temperature of about 50° until frothing took place. Upon completion of the frothing, 200ml of buffer was added to replace the water lost during evaporation. The liposomes just formed were left in a water bath at 50°C for 30 minutes, after which they were cooled rapidly to ambient temperature in an ice bath and dialysed for 24 hours against a 300 mOsM HEPES buffer to eliminate the non-encapsulated active ingredient and indocyanine green.

The heat-sensitive liposomes thus obtained entrapped about 30% of the available cytosine arabinoside and indocyanine green, had an average diameter of about 200nm, and were stable up to 41°C.

A suspension of liposomes thus obtained in an equal volume of bovine foetal serum was irradiated with a diode laser with a power of 3W and a wavelength of 805nm for about 10 seconds, thus raising the temperature to 45°C.

The indocyanine green liberated was determined photometrically and it was found that the diode laser irradiation had led to quantitative release of the indocyanine green contained in the liposomes.

This also meant that the structure of the liposomes had yielded under the effect of the temperature rise induced by the excitation of the indocyanine green by means of the laser radiation.

Discharge of the cytosine arabinoside occurred simultaneously with the release of the indocyanine.

Since the liposomes produced in accordance with this example were found stable in serum at normal body temperature, the possibility arises of using these liposomes in pharmaceutical compositions for the treatment of localized conditions by the administration of these compositions by a systemic (for example, intravenous) route and the subjection solely of the region affected by the condition to laser radiation.

### EXAMPLE 2

### Cream for treating cellulite

0.5kg of caffeine was dissolved, together with 1.0kg of indocyanine green and 0.10 kg of bisabolol in 45kg of 10mM phosphate buffer at pH 7.0 in an emulsifier with a stirrer.

When dissolving had been achieved, 0.6kg of purified granular soya lecithin was added with stirring. The resulting suspension was kept at 20°C with uniform stirring for a period of 2-4 hours so as to bring about the formation of the liposomes and was then transferred into a stainless steel container and kept at 4°C until the moment when the cream was prepared.

Separately, a substrate gel was prepared by dissolving 0.15kg of propylparaben, 0.15kg of methylparaben, 3.0kg of glycerol and 0.2kg of bromonitropropandiol in 46kg of a 1% solution of neutralized Carbapol 934. From 0.5kg to 2.0kg of Carbapol 934 was added according to the desired density.

The thickened solution thus produced was kept at ambient temperature until the moment when the cream was prepared.

The liposome suspension was transferred into the emulsifier and the substrate gel was added thereto with intense stirring (approximately 1000 revolutions per minute) over about 60 minutes at a temperature of 20° and at a pressure of 400mm Hg, to give the desired cream.

The cream thus prepared can be used to treat cellulite in accordance with the method of the invention, that is, by its application to the regions of the body affected, followed by irradiation of the application sites with a diode laser having a power of 3W and a wavelength of 780-800nm. Irradiation normally continues for about ten seconds and affects an area of about 2cm² of skin.

In this case also, the liposomes act as extremely efficient vectors for the active ingredients, enabling the dermis and even the subcutaneous tissues and, in particular, the adipocytes, to be reached.

Subsequent laser irradiation brings about absorption of energy by the indocyanine green and a consequent local temperature rise with the release of the active ingredient and amplification of its effects on the adipocytes. The rise in temperature which occurs in the vicinity of the adipocytes also contributes to the lysis of these cell structures.

Naturally, the liposomes according to the invention may contain various active ingredients having either therapeutic or cosmetic activity.

Purely by way of indication, some of the active ingredients which may be incorporated in the liposomes and in the compositions according to the invention may be listed; amongst these, both steroidal and non-steroidal anti-inflammatories, antibiotics, anti-fungal agents, anti-tumour agents, antipyretics, AHAs, vitamin complexes, antioxidants, hormones, collagens, elastin, glycolic acid, water, oxygen, etc., may be mentioned.

Finally, it is possible to increase the selectivity of the treatments with the use of the liposomes according to the invention by binding the latter to monoclonal antibodies which can selectively recognize surface antigens present on the cytoplasmic membranes of the target cells.

### EXAMPLE 3

A gel having the following composition was prepared:

| | |
|---|---|
| mannitol | 6.00% |
| glycolic acid | 5.00% |
| indocyanine green | 0.25% |
| acrylate/C10-C30 alkylacryl. crosspol. | 0.20% |
| disodium EDTA | 0.10% |
| sodium lauryl sulphate | 0.05% |
| allantoin | 0.01% |
| pantenol | 0.01% |
| sodium hydroxide | 0.11% |
| methylparaben | 0.20% |
| water to make up to | 100.00% |

The gel thus prepared succeeds in transporting the active ingredient (glycolic acid) quickly and deeply by virtue of the raised temperature brought about by the excitation of the indocyanine green as a result of the irradiation of the region of application with the diode laser. This is in addition to the characteristic vasodilatory and biostimulating effects of the infra-red radiation.

The effect of the glycolic acid is thus considerably accelerated and amplified in comparison with that which can be achieved by conventional cosmetic formulations.

## Claims

1. Liposomes containing at least one pharmacologically or cosmetically active ingredient, characterized in that they further contain at least one photosensitizing substance showing a high degree of absorption at at least one wavelength of a light source.

2. Liposomes according to Claim 1, characterized in that the photosensitizing substance is selected from the group comprising haematoporphyrin derivatives, chlorophyll derivatives, indocyanine green, photophrin II, micro-cyanine, and Chlorin-E6, and the light source is a laser beam.

3. Liposomes according to Claim 2, characterized in that the photosensitizing substance is indocyanine green and the laser beam is emitted by a continuous or micro-pulsed power diode laser with a wavelength of 700-900nm, preferably 780-820nm.

4. A cosmetic composition for topical application comprising a cosmetically acceptable vehicle and liposomes according to any one of the preceding claims.

5. A cosmetic composition for topical application according to Claim 4, comprising liposomes according to Claim 3.

6. A cosmetic treatment method comprising the topical application of a composition according to Claim 4 or Claim 5 and the subsequent irradiation of the application site with a source of light having a wavelength at which the photosensitizing substance shows absorption.

7. A pharmaceutical composition comprising a pharmaceutically acceptable excipient and liposomes according to any one of Claims 1 to 3.

8. A pharmaceutical composition according to Claim 7, comprising liposomes according to Claim 3.

9. A pharmaceutical composition according to Claim 7 or Claim 8 for use in a therapeutic treatment method comprising the topical application of the composition and the subsequent irradiation of the application site with a source of light having a wavelength at which the photosensitizing substance shows absorption.

10. A pharmaceutical composition according to Claim 7 or Claim 8 for use in a therapeutic treatment method comprising the administration of the composition by a systemic route and the subsequent irradiation of one or more anatomical regions which require such treatment with a source of light having a wavelength at which the photosensitizing substance shows absorption.

11. Use of liposomes according to any one of Claims 1-3 for the cosmetic treatment of the human body.

12. A cosmetic composition for topical application comprising a cosmetically acceptable vehicle, at least one cosmetically active substance, and at least one photosensitizing substance showing a high degree of absorption at at least one wavelength of a light source.

13. A cosmetic composition according to Claim 12, characterized in that the photosensitizing substance is selected from the group comprising haematoporphyrin derivatives, chlorophyll derivatives, indocyanine green, photophrin II, micro-cyanine, and Chlorin-E6, and the light source is a laser beam.

14. A cosmetic composition according to Claim 13, characterized in that the photosensitizing substance is indocyanine green and the laser beam is emitted by a continuous or micro-pulsed power diode laser with a wavelength of 700-900nm, preferably 780-820nm.

15. A cosmetic composition according to Claim 14, in which the quantity of indocyanine green contained is variable from 0.01 to 10.0% by weight, preferably from 0.05-5.0% by weight.

16. A cosmetic treatment method comprising the topical application of a composition according to any one of Claims 12 to 15 and the subsequent irradiation of the application site with a light source having a wavelength at which the photosensitizing substance shows absorption.
